# EUROPÄISCHE PATENTSCHRIFT

(11) **EP 0 876 326 B1**
(45) Veröffentlichungstag und Bekanntmachung des Hinweises auf die Patenterteilung: **04.04.2001**
(21) Anmeldenummer: 96942337.5
(22) Anmeldetag: 04.12.1996
(51) Int. Cl.: C07C 209/60, C07C 211/07

(54) **VERFAHREN ZUR HERSTELLUNG VON AMINEN AUS OLEFINEN AN ZEOLITHEN DES TYPS SSZ-26, SSZ-33, CIT-1 ODER DEREN GEMISCHEN**
PROCESS FOR PRODUCING AMINES FROM OLEFINS ON ZEOLITHS OF TYPES SSZ-26, SSZ-33, CIT-1 OR MIXTURES THEREOF
PROCEDE DE PRODUCTION D'AMINES A PARTIR D'OLEFINES SUR ZEOLITHES DU TYPE SSZ-26, SSZ-33, CIT-1 OU DES MELANGES DE CES DERNIERES

(30) Priorität: 08.12.1995 DE 19545875
(43) Veröffentlichungstag der Anmeldung: 11.11.1998
(73) Patentinhaber: BASF AKTIENGESELLSCHAFT, 67056 Ludwigshafen (DE)
(72) Erfinder: ELLER, Karsten, D-67059 Ludwigshafen (DE); KUMMER, Rudolf, D-67227 Frankenthal (DE); STOPS, Peter, D-67122 Altrip (DE)
(86) Internationale Anmeldenummer: EP9605406
(87) Internationale Veröffentlichungsnummer: WO9721661

(56) Entgegenhaltungen:
- CA-A- 2 092 964

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drükken in Gegenwart von Zeolithen des Typs SSZ-26, SSZ-33, CIT-1 oder deren Gemischen.

Eine Übersicht über die Methoden zur Aminierung von Olefinen wird in "Functionalisation of Alkenes: Catalytic Amination of Monoolefins", J.J. Brunet et al. J. Mol. Catal., 49 (1989), Seiten 235 bis 259 gegeben.

Grundsätzlich gibt es zwei Katalysemechanismen. Das Olefin wird über einen Metallkomplex koordiniert. Diese aktivierte Spezies kann von dem nucleophilen Amin angegriffen werden und ein höher aminiertes Produkt bilden. Das Amin kann an Säurezentren oder an Metallzentren (via Metallamide) chemisorbiert werden und so aktiviert mit dem Olefin umgesetzt werden.

Gut geeignete Katalysatoren sind Zeolithe. Sie zeichnen sich durch eine hohe Anzahl an katalytisch aktiven Zentren aus, kombiniert mit einer großen Oberfläche. Die beschriebenen Zeolithe unterscheiden sich im Typ und in der Nachbehandlung (z.B. thermische Behandlung, Dealuminierung, Säurebehandlung, Metallioneneintausch, etc.). Beispiele hierfür finden sich in US-A-4 375 002, US-A-4 536 602, EP-A-305 564, EP-A-101 921, DE-A-42 06 992.

Aus EP-A-133 938, EP-A-431 451 und EP-A-132 736 sind Verfahren bekannt, bei denen Bor-, Gallium-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Å definiert sind, eingesetzt werden. Bevorzugt werden metall- oder halogenmodifizierte Beta-Zeolithe eingesetzt.

Alle Verfahren zur Synthese von Aminen aus Olefinen an diesen Katalysatoren zeichnen sich durch eine geringe Amin-Ausbeute oder geringe Raum-Zeit-Ausbeute aus, oder führen zu einer raschen Desaktivierung der Katalysatoren.

Der vorliegenden Erfindung lag daher die Aufgabe zugrunde, diesen Nachteilen abzuhelfen.

Demgemäß wurde ein neues und verbessertes Verfahren zur Herstellung von Aminen der allgemeinen Formel I 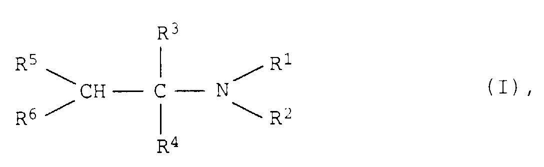 in der
- R¹,R²,R³,R⁴,R⁵,R⁶: Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl bedeuten, und
- R¹ und R²: gemeinsam eine gesättigte oder ungesättigte C₃-bis C₉-Alkylendikette und
- R³ oder R⁵: C₂₁- bis C₂₀₀-Alkyl, C₂₁- bis C₂₀₀-Alkenyl oder gemeinsam eine C₂- bis C₁₂-Alkylendikette
zusätzlich bedeuten können, durch Umsetzung von Olefinen der allgemeinen Formel II 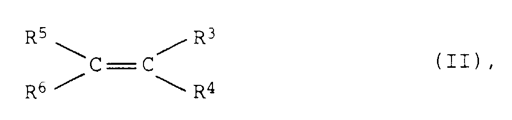 in der R³, R⁴, R⁵ und R⁶ die oben genannten Bedeutungen haben, mit Ammoniak oder primären oder sekundären Aminen der allgemeinen Formel III 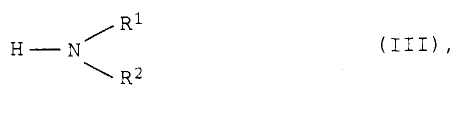 in der R¹ und R² die oben genannten Bedeutungen haben, bei Temperaturen von 200 bis 350°C und Drücken von 100 bis 300 bar in Gegenwart eines Heterogenkatalysators gefunden, welches dadurch gekennzeichnet ist, daß man als Heterogenkatalysator Zeolithe des Typs SSZ-26, SSZ-33, CIT-1 oder deren Gemische einsetzt.

Das erfindungsgemäße Verfahren kann wie folgt durchgeführt werden:

Das Olefin II und Ammoniak oder das primäre oder sekundären Amin III kann bei Temperaturen von 200 bis 350°C, bevorzugt 220 bis 330°C, besonders bevorzugt 230 bis 320°C und Drücken von 100 bis 300 bar, bevorzugt 120 bis 300 bar, besonders bevorzugt 140 bis 290 bar in Gegenwart von Zeolithen des Typs SSZ-26, SSZ-33, CIT-1 oder deren Gemischen als Katalysator z.B. in einem Druck-Reaktor umsetzt werden, und bevorzugt das erhaltene Amin abgetrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung des Katalysators zurückgedrängt worden.

Das erfindungsgemäße Verfahren zeichnet sich dadurch aus, daß bereits mit niedrigem Ammoniak- bzw. Amin- Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Als Katalysatoren für die Aminierung von Olefinen eignen sich Zeolithe des Typs SSZ-26, SSZ-33, CIT-1 oder deren Gemische. Die Präparation von SSZ-26 wird in US-A-4 910 006 beschrieben, diejenige von SSZ-33 in US-A-4 963 337, US-A-5 120 425 und WO-A-94/00233 und die von CIT-1 in WO-A-95/07859. Wie aus Stud. Surf. Sci. Catal., 84, 461 bis 468 (1994) bekannt, sind das Alumosilikat SSZ-26 und die beiden Borosilikate SSZ-33 und CIT-1 strukturell sehr nahe verwandt und unterscheiden sich nur durch die Stapelabfolge zweier verschiedener Polymorphe.

Die erfindungsgemäßen Zeolithe SSZ-26, SSZ-33 und CIT-1 können als solche verformt werden, oder aber auch mit einem Bindemittel im Verhältnis 98:2 bis 40:60 Gew.-% zu Strängen oder Tabletten. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Preßlinge zweckmäßig bei 110°C/16 h getrocknet und bei 200 bis 500°C/2 bis 16 h calciniert, wobei die Calcinierung auch direkt im Aminierungsreaktor erfolgen kann.

Zur Erhöhung der Selektivität, der Standzeit und der Anzahl der möglichen Regenerierungen kann man verschiedene Modifizierungen an den erfindungsgemäßen Zeolithkatalysatoren SSZ-26, SSZ-33 und CIT-1 vornehmen.

Eine Modifizierung der Katalysatoren besteht darin, daß man die unverformten oder die verformten Zeolithe mit Alkalimetallen wie Na und K, Erdalkalimetallen wie Ca, Mg, Erdmetallen wie Tl, Übergangsmetallen wie z.B. Ti, Zr, Mn, Fe, Mo, Cu, Zn, Cr, Edelmetallen und/oder seltenen Erdmetallen wie z.B. La, Ce oder Y ionenaustauschen bzw. dotieren kann.

Eine vorteilhafte Ausführungsform besteht darin, daß man die verformten erfindungsgemäßen Zeolithe SSZ-26, SSZ-33 und CIT-1 in einem Strömungsrohr vorlegt und bei 20 bis 100°C z.B. ein Halogenid, ein Acetat, ein Oxalat, ein Citrat oder ein Nitrat der oben beschriebenen Metalle in gelöster Form darüberleitet. Ein derartiger Ionenaustausch kann z.B. an der Wasserstoff-, Ammonium- und Alkaliform der erfindungsgemäßen Zeolithe SSZ-26, SSZ-33 und CIT-1 vorgenommen werden.

Eine weitere Möglichkeit der Metallaufbringung auf die erfindungsgemäßen Zeolithe SSZ-26, SSZ-33 und CIT-1 besteht darin, daß man das Material z.B. mit einem Halogenid, einem Acetat, einem Oxalat, einem Citrat, einem Nitrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten Zeolithen des Typs SSZ-26, SSZ-33 und CIT-1 kann eine Nachbehandlung mit Wasserstoff und/ oder mit Wasserdampf günstig sein.

Eine weitere Möglichkeit der Modifizierung besteht darin, daß man die erfindungsgemäßen Zeolithe SSZ-26, SSZ-33 und CIT-1-verformt oder unverformt - einer Behandlung mit Säuren, wie Salzsäure (HCl), Flußsäure (HF), Schwefelsäure (H₂SO₄), Oxalsäure (HO₂C-CO₂H), Phosphorsäure (H₃PO₄) oder deren Gemischen unterwirft.

Eine besondere Ausführungsform besteht darin, daß man die erfindungsgemäßen Zeolithe SSZ-26, SSZ-33 und CIT-1 vor der Verformung mit einer der genannten Säuren 0,001 n bis 2 n, bevorzugt 0,05 bis 0,5 n 1 bis 100 Stunden unter Rückfluß behandelt. Nach Abfiltrieren und Auswaschen wird in der Regel bei 100 bis 160°C getrocknet und bei 200 bis 600°C calciniert. Eine weitere besondere Ausführungsform liegt in einer Säure-Behandlung der erfindungsgemäßen Zeolithe SSZ-26, SSZ-33 und CIT-1 nach ihrer Verformung mit Bindemittel. Hierbei wird der erfindungsgemäße Zeolith in der Regel 1 bis 3 Stunden zwischen 60 und 80°C mit einer 3 bis 25%igen, insbesondere mit einer 12 bis 20%igen Säure behandelt, anschließend ausgewaschen, bei 100 bis 160°C getrocknet und bei 200 bis 600°C calciniert. Auch hier kann die Calcinierung direkt im Aminierungsreaktor erfolgen.

Eine andere Möglichkeit der Modifizierung ist gegeben durch einen Austausch mit Ammonsalzen, z.B. mit NH₄Cl oder mit Mono-, Di- oder Polyaminen. Hierbei wird der mit Bindemittel verformte Zeolith in der Regel bei 60 bis 80°C mit 10 bis 25%iger, bevorzugt 20%iger NH₄Cl-Lösung 2 h kontinuierlich in gewichtsmäßiger Zeolith/Ammonchlorid-Lösung von 1:15 ausgetauscht und danach bei 100 bis 120°C getrocknet.

Eine weitere Modifikation, die an den erfindungsgemäßen Zeolithen vorgenommen werden kann, ist die Dealuminierung im Falle des Aluminium-Zeolithen SSZ-26, bei der ein Teil der Aluminumatome durch Silicium ersetzt wird oder die Zeolithe durch beispielsweise hydrothermale Behandlung in ihrem Aluminumgehalt abgereichert werden. An eine hydrothermale Dealuminierung schließt sich vorteilhafterweise eine Extraktion mit Säuren oder Komplexbildnern an, um gebildetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen von Y-Zeolithen finden sich in Corma et al., Stud. Surf. Sci. Catal. 37 (1987), Seiten 495 bis 503. Im Falle des Bor-Zeolithe SSZ-33 und CIT-1 kann entsprechend ein Teil des Bors herausgelöst und durch Silicium ersetzt werden.

Die Katalysatoren kann man ais Stränge mit Durchmessern von z.B. 1 bis 4 mm oder als Tabletten mit z.B. 3 bis 5 mm Durchmesser für die Aminierung der Olefine einsetzen.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
R¹,R²,R³,R⁴,R⁵,R⁶
   - Wasserstoff,
   - C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
   - C₂- bis C₂₀-Alkenyl, bevorzugt C₂- bis C₁₂-Alkenyl, besonders bevorzugt C₂- bis C₈-Alkenyl wie Vinyl und Allyl,
   - C₂- bis C₂₀-Alkinyl, bevorzugt C₂- bis C₈-Alkinyl, insbesondere C₂H und Propargyl,
   - C₃- bis C₂₀-Cycloalkyl, bevorzugt C₃- bis C₁₂-Cycloalkyl, besonders bevorzugt C₅- bis C₈-Cycloalkyl wie Cyclopentyl, Cyclohexyl, Cycloheptyl und Cyclooctyl,
   - C₄- bis C₂₀-Alkyl-cycloalkyl, bevorzugt C₄- bis C₁₂-Alkyl-cycloalkyl, besonders bevorzugt C₅- bis C₁₀-Alkyl-cycloalkyl,
   - C₄- bis C₂₀-Cycloalkyl-alkyl, bevorzugt C₄- bis C₁₂-Cycloalkyl-alkyl, besonders bevorzugt C₅- bis C₁₀-Cycloalkyl-alkyl,
   - Aryl wie Phenyl, 1-Naphthyl und 2-Naphthyl, bevorzugt Phenyl,
   - C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
   - C₇- bis C₂₀-Aralkyl, bevorzugt C₇- bis C₁₆-Aralkyl, bevorzugt C₇- bis C₁₂-Phenalkyl wie Phenylmethyl, 1-Phenylethyl, 2-Phenylethyl,
R¹ und R²
   - gemeinsam eine gesättigte oder ungesättigte C₃- bis C₉-Alkylendikette, bevorzugt -(CH₂)₄-, -(CH₂)₅-, -(CH₂)₇- und
   - CH=CH-CH=CH-,
R³ oder R⁵
   - C₂₁- bis C₂₀₀-Alkyl, bevorzugt C₄₀- bis C₂₀₀-Alkyl, wie Polybutyl, Polyisobutyl, Polypropyl, Polyisopropyl und Polyethyl, besonders bevorzugt Polybutyl und Polyisobutyl,
   - C₂₁- bis C₂₀₀-Alkenyl, bevorzugt C₄₀- bis C₂₀₀-Alkenyl, besonders bevorzugt C₇₀- bis C₁₇₀-Alkenyl,
R³ und R⁵
   - gemeinsam eine C₂- bis C₁₂-Alkylendikette, bevorzugt eine C₃-bis C₈-Alkylendikette, besonders bevorzugt -(CH₂)₃-, -(CH₂)₄-, -(CH₂)₅-, -(CH₂)₆- und -(CH₂)₇-, insbesondere - (CH₂)₃- und - (CH₂)₄-.

### Beispiele

### Katalysatorsynthese

### Katalysator A

30g SSZ-26 wurden mit 20g Boehmit und 1g Ameisensäure versetzt, im Kneter kompaktiert und unter Wasserzusatz (65 ml) 45 Minuten verknetet. In einer Strangpresse wurden mit einem Preßdruck von 40 bar 2mm Stränge erzeugt, 16 h bei 120°C getrocknet und an- schließend 16 h bei 500°C calciniert.

### Katalysator B

30g SSZ-33 wurden mit 20g Boehmit und 1g Ameisensäure versetzt, im Kneter kompaktiert und unter Wasserzusatz (49 ml) 45 Minuten verknetet. In einer Strangpresse wurden mit einem Preßdruck von 40 bar 2mm Stränge erzeugt, 16 h bei 120°C getrocknet und anschließend 16 h bei 500°C calciniert.

### Katalysator C

30g CIT-1 wurden mit 20g Boehmit und 1g Ameisensäure versetzt, im Kneter kompaktiert und unter Wasserzusatz (67 ml) 45 Minuten verknetet. In einer Strangpresse wurden mit einem Preßdruck von 40 bar 2mm Stränge erzeugt, 16 h bei 120°C getrocknet und an- schließend 16 h bei 500°C calciniert.

### Aminierungsbeispiele

Die Versuche wurden in einem Rohrreaktor (6 mm Innendurchmesser) unter isothermen Bedingungen bei 260 bis 300°C und einem Druck von 280 bar mit einem Gemisch aus Isobuten und Ammoniak im molaren Verhältnis von 1:1,5 durchgeführt. Die Reaktionsprodukte wurden im Gaschromatographen analysiert.

Die Ergebnisse sind in Tabelle 1 zusammengestellt.

**Tabelle 1**

| tert.-Butylamin (NH₃: C₄H₈ = 1,5) | | | | | | |
|---|---|---|---|---|---|---|
| Katalysator | Druck | Temperatur | tert.-Butylamin-Ausbeute [Gew.-%] | | | Litergewicht |
| | [bar] | [°C] | WHSV 0,7 [g/g · h] | WHSV 1,5 [g/g · h] | WHSV 3 [g/g · h] | [kg/l] |
| A | 280 | 260 | 19,2 | | | 0,48 |
| A | 280 | 270 | 20,9 | 18,2 | 14,6 | 0,48 |
| A | 280 | 280 | 18,0 | 17,5 | 15,2 | 0,48 |
| A | 280 | 300 | | | 12,6 | 0,48 |
| B | 280 | 290 | 17,7 | 17,0 | 13,5 | 0,52 |
| C | 280 | 275 | 19,2 | 18,0 | 15,0 | 0,50 |

## Patentansprüche

1. Verfahren zur Herstellung von Aminen der allgemeinen Formel I 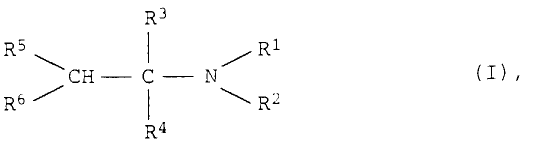 in der
R¹,R²,R³,R⁴,R⁵,R⁶ Wasserstoff, C₁- bis C₂₀-Alkyl, C₂- bis
C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇-bis C₂₀-Alkylaryl oder C₇- bis C₂₀-Aralkyl bedeuten, und
R¹ und R² gemeinsam eine gesättigte oder C₃- bis C₉-Alkylendikette undungesättigte
R³ oder R⁵ C₂₁- bis C₂₀₀-Alkyl, C₂₁- bis C₂₀₀-Alkenyl oder gemeinsam eine C₂- bis C₁₂-Alkylendikette
zusätzlich bedeuten können, durch Umsetzung von Olefinen der allgemeinen Formel II 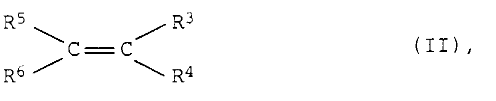 in der R³, R⁴, R⁵ und R⁶ die oben genannten Bedeutungen haben, mit Ammoniak oder primären oder sekundären Aminen der allgemeinen Formel III 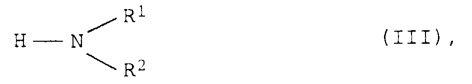 in der R¹ und R² die oben genannten Bedeutungen haben, bei Temperaturen von 200 bis 350°C und Drücken von 100 bis 300 bar in Gegenwart eines Heterogenkatalysators, dadurch gekennzeichnet, daß man als Heterogenkatalysator Zeolithe des Typs SSZ-26, SSZ-33, CIT-1 oder deren Gemische einsetzt.

2. Verfahren zur Herstellung von Aminen I nach Anspruch 1, dadurch gekennzeichnet, daß man das gebildete Amin I abtrennt und die nichtumgesetzten Einsatzstoffe II und III zurückführt.

3. Verfahren zur Herstellung von Aminen nach den Ansprüchen 1 bis 2, dadurch gekennzeichnet, daß man als Olefin II Isobuten, Diisobuten, Cyclopenten, Cyclohexen oder Polyisobuten einsetzt.

4. Verfahren zur Herstellung von Aminen nach den Ansprüchen 1 bis 3, dadurch gekennzeichnet, daß man als Heterogenkatalysatoren Zeolithe des Typs SSZ-26, SSZ-33, CIT-1 oder deren Gemische in der H-Form einsetzt.

5. Verfahren zur Herstellung von Aminen nach den Ansprüchen 1 bis 4, dadurch gekennzeichnet, daß man als Heterogenkatalysatoren Zeolithe des Typs SSZ-26, SSZ-33, CIT-1 oder deren Gemische, die mit einer Säure, insbesondere einer aus der Gruppe Salzsäure, Flußsäure, Schwefelsäure, Oxalsäure, Phosphorsäure oder deren Gemischen, behandelt sind, einsetzt.

6. Verfahren zur Herstellung von Aminen nach den Ansprüchen 1 bis 5, dadurch gekennzeichnet, daß man als Heterogenkatalysatoren Zeolithe des Typs SSZ-26, SSZ-33, CIT-1 oder deren Gemische, die mit einem oder mehreren Übergangsmetallen dotiert sind, einsetzt.

7. Verfahren zur Herstellung von Aminen nach den Ansprüchen 1 bis 6, dadurch gekennzeichnet, daß man als Heterogenkatalysatoren Zeolithe des Typs SSZ-26, SSZ-33, CIT-1 oder deren Gemische, die mit einem oder mehreren Elementen der Seltenen Erden dotiert sind, einsetzt.

8. Verfahren zur Herstellung von Aminen nach den Ansprüchen 1 bis 7, dadurch gekennzeichnet, daß man als Heterogenkatalysatoren Zeolithe des Typs SSZ-26, SSZ-33, CIT-1 oder deren Gemische in der Ammoniumform einsetzt.

9. Verfahren zur Herstellung von Aminen nach den Ansprüchen 1 bis 8, dadurch gekennzeichnet, daß man als Heterogenkatalysatoren Zeolithe des Typs SSZ-26, SSZ-33, CIT-1 oder deren Gemische einsetzt, die mit einem oder mehreren Elementen aus der Gruppe der Alkali-, Erdalkalimetalle oder Erdmetalle dotiert sind.

10. Verfahren zur Herstellung von Aminen nach den Ansprüchen 1 bis 9, dadurch gekennzeichnet, daß man als Heterogenkatalysatoren Zeolithe des Typs SSZ-26, SSZ-33, CIT-1 oder deren Gemische einsetzt, die mit einem Bindemittel verformt und bei Temperaturen von 200 bis 600°C calciniert sind.

11. Verfahren zur Herstellung von Aminen nach den Ansprüchen 1 bis 10. dadurch gekennzeichnet, daß man als Heterogenkatalysatoren dealuminierte oder deborierte Zeolithe des Typs SSZ-26, SSZ-33, CIT-1 oder deren Gemische einsetzt.

## Claims

1. A process for preparing amines of the general formula I 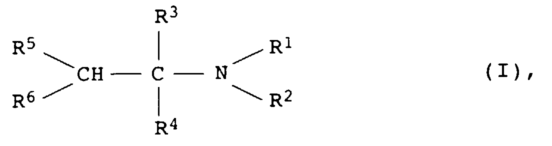 where
R¹,R²,R³,R⁴,R⁵ and R⁶ are each hydrogen, C₁-C₂₀-alkyl, C₂-C₂₀-alkenyl, C₂-C₂₀-alkynyl, C₃-C₂₀-cycloalkyl, C₄-C₂₀-alkylcycloalkyl, C₄-C₂₀-cycloalkylalkyl, aryl, C₇-C₂₀-alkylaryl or C₇-C₂₀-aralkyl, and, additionally,
R¹ and R² together may be a saturated or unsaturated C₃-C₉-alkylene dichain, and
R³ or R⁵ may each be C₂₁-C₂₀₀-alkyl or C₂₁-C₂₀₀-alkenyl or together a C₂-C₁₂-alkylene dichain,
by reacting olefins of the general formula II 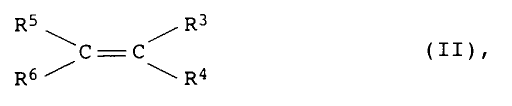 where R³, R⁴, R⁵ and R⁶ are each as defined above, with ammonia or primary or secondary amines of the general formula III 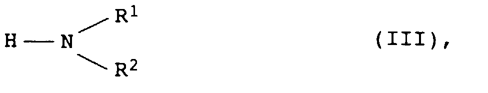 where R¹ and R² are each as defined above, at temperatures from 200 to 350°C and pressures from 100 to 300 bar in the presence of a heterogeneous catalyst, which comprises using a heterogeneous catalyst comprising zeolites of the type SSZ-26, SSZ-33, CIT-1 or mixtures thereof.

2. A process for preparing amines I as claimed in claim 1, wherein the product amine I is separated off and the unconverted feed materials II and III are recycled.

3. A process for preparing amines as claimed in claim 1 or 2, wherein olefin II is isobutene, diisobutene, cyclopentene, cyclohexene or polyisobutene.

4. A process for preparing amines as claimed in any of claims 1 to 3, wherein the heterogeneous catalyst used comprises a zeolite of the type SSZ-26, SSZ-33, CIT-1 or a mixture thereof in the H-form.

5. A process for preparing amines as claimed in any of claims 1 to 4, wherein the heterogeneous catalyst used comprises a zeolite of the type SSZ-26, SSZ-33, CIT-1 or a mixture thereof which has been treated with an acid, in particular with an acid selected from the group consisting of hydrochloric acid, hydrofluoric acid, sulfuric acid, oxalic acid, phosphoric acid or mixtures thereof.

6. A process for preparing amines as claimed in any of claims 1 to 5, wherein the heterogeneous catalyst used comprises a zeolite of the type SSZ-26, SSZ-33, CIT-1 or a mixture thereof doped with one or more transition metals.

7. A process for preparing amines as claimed in any of claims 1 to 6, wherein the heterogeneous catalyst used comprises a zeolite of the type SSZ-26, SSZ-33, CIT-1 or a mixture thereof doped with one or more rare earth elements.

8. A process for preparing amines as claimed in any of claims 1 to 7, wherein the heterogeneous catalyst used comprises a zeolite of the type SSZ-26, SSZ-33, CIT-1 or a mixture thereof in the ammonium form.

9. A process for preparing amines as claimed in any of claims 1 to 8, wherein the heterogeneous catalyst used comprises a zeolite of the type SSZ-26, SSZ-33, CIT-1 or a mixture thereof doped with one or more elements from the group of the alkali, alkaline earth or earth metals.

10. A process for preparing amines as claimed in any of claims 1 to 9, wherein the heterogeneous catalyst used comprises a zeolite of the type SSZ-26, SSZ-33, CIT-1 or a mixture thereof molded with a binder and calcined at temperatures from 200 to 600°C.

11. A process for preparing amines as claimed in any of claims 1 to 10, wherein the heterogeneous catalyst used comprises a dealuminated or deborated zeolite of the type SSZ-26, SSZ-33, CIT-1 or a mixture thereof.

## Revendications

1. Procédé de préparation d'amines de la formule générale I 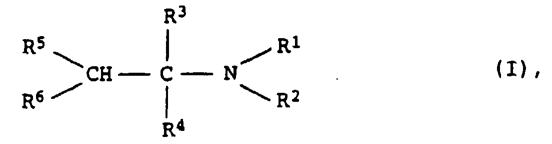 dans laquelle
R¹, R², R³, R⁴, R⁵, R⁶ peuvent représenter en outre l'hydrogène, ou un radical alkyle en C₁ à C₂₀, alcényle en C₂ à C₂₀, alkinyle en C₂ à C₂₀, cycloalkyle en C₃ à C₂₀, alkylcycloalkyle en C4 à C₂₀, cycloalkylalkyle en C₄ à C₂₀, aryle, alkylaryle en C₇ à C₂₀ ou aralkyle en C₇ à C₂₀ et
R¹ et R² peuvent représenter ensemble une double chaîne alkylénique saturée ou insaturée en C₃ à C₉ et
R³ ou R⁵ peuvent représenter un radical alkyle en C₂₁ à C₂₀₀, alcényle en C₂₁ à C₂₀₀, ou représenter ensemble une double chaîne alkylénique en C₂ à C₁₂
par réaction d'oléfines de la formule générale II 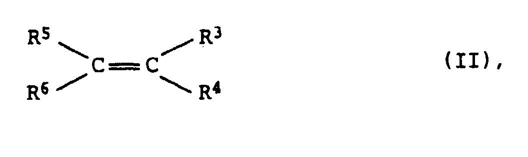 dans laquelle R³, R⁴, R⁵ et R⁶ ont les significations ci-dessus, avec de l'ammoniac ou des amines primaires ou secondaires de la formule générale III 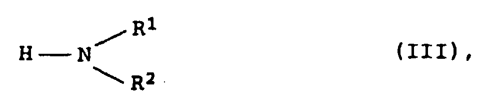 dans laquelle R¹ et R² ont les significations ci-dessus, à des températures de 200 à 350°C et des pressions de 100 à 300 bar, en présence d'un catalyseur hétérogène, caractérisé en ce que l'on met en oeuvre, en tant que catalyseur hétérogène, des zéolithes du type SSZ-26, SSZ-33, CIT-1 ou leurs mélanges.

2. Procédé de préparation d'amines I selon la revendication 1, caractérisé en ce que l'on sépare l'amine I formée et l'on recycle les substances de départ II et III n'ayant pas réagi.

3. Procédé de préparation d'amines selon les revendications 1 à 2, caractérisé en ce que l'on met en oeuvre, en tant qu'oléfine II, de l'isobutène, du diisobutène, du cyclopentène, du cyclohexène ou du polyisobutène.

4. Procédé de préparation d'amines selon les revendications 1 à 3, caractérisé en ce que l'on met en oeuvre, en tant que catalyseurs hétérogènes, des zéolithes de type SSZ-26, SSZ-33, CIT-1, ou leurs mélanges, sous leur forme H.

5. Procédé de préparation d'amines selon les revendications 1 à 4, caractérisé en ce que l'on met en oeuvre, en tant que catalyseurs hétérogènes, des zéolithes de type SSZ-26, SSZ-33, CIT-1, ou leurs mélanges, après traitement à l'acide, en particulier des acides choisis dans le groupe formé par l'acide chlorhydrique, l'acide fluorhydrique, l'acide sulfurique, l'acide oxalique, l'acide phosphorique ou leurs mélanges.

6. Procédé de préparation d'amines selon les revendications 1 à 5, caractérisé en ce que l'on met en oeuvre, comme catalyseurs hétérogènes, des zéolithes de type SSZ-26, SSZ-33, CIT-1, ou leurs mélanges, après dopage par un ou plusieurs métaux de transition.

7. Procédé de préparation d'amines selon les revendications 1 à 6, caractérisé en ce que l'on met en oeuvre, en tant que catalyseurs hétérogènes, des zéolithes de type SSZ-26, SSZ-33, CIT-1, ou leurs mélanges, après dopage par un ou plusieurs éléments parmi les terres rares.

8. Procédé de préparation d'amines selon les revendications 1 à 7, caractérisé en ce que l'on met en oeuvre, en tant que catalyseurs hétérogènes, des zéolithes de type SSZ-26, SSZ-33, CIT-1, ou leurs mélanges, sous leur forme ammoniacale.

9. Procédé de préparation d'amines selon les revendications 1 à 8, caractérisé en ce que l'on met en oeuvre, en tant que catalyseurs hétérogènes, des zéolithes de type SSZ-26, SSZ-33, CIT-1, ou leurs mélanges, après dopage par un ou plusieurs éléments des groupes des métaux alcalins et alcalino-terreux ou des métaux terreux.

10. Procédé de préparation d'amines selon les revendications 1 à 9, caractérisé en ce que l'on met en oeuvre, en tant que catalyseurs hétérogènes, des zéolithes de type SSZ-26, SSZ-33. CIT-1, ou leurs mélanges, après formage avec un liant et calcination à des températures de 200 à 600°C.

11. Procédé de préparation d'amines selon les revendications 1 à 10, caractérisé en ce que l'on utilise comme catalyseurs des zéolithes désaluminées ou déborées de type SSZ-26, SSZ-33, CIT-1 ou leurs mélanges.
